# FASCICULE DE BREVET EUROPEEN

(11) **EP 0 929 512 B1**
(45) Date de publication et mention de la délivrance du brevet: **05.12.2001**
(21) Numéro de dépôt: 97940226.0
(22) Date de dépôt: 12.09.1997
(51) Int. Cl.: C07C 57/04, C07C 51/487, C07C 51/50, C07C 51/44

(54) **PROCEDE PERFECTIONNE DE PURIFICATION DE L'ACIDE ACRYLIQUE**
VERFAHREN ZUR REINIGUNG DER ACRYLSÄURE
IMPROVED METHOD FOR PURIFYING ACRYLIC ACID

(30) Priorité: 16.09.1996 FR 9611269
(43) Date de publication de la demande: 21.07.1999
(73) Titulaire: Atofina, 92800 Puteaux (FR)
(72) Inventeur: FAUCONET, Michel, F-57330 Valmont (FR); AUGUSTIN, Francis, F-57260 Lindre Basse (FR); ESCH, Marc, F-57800 Freyming Merlebach (FR)
(74) Mandataire: Chaillot, Geneviève
(86) Numéro de dépôt international: FR9701613
(87) Numéro de publication internationale: WO9811048

(56) Documents cités:
- EP-A- 0 085 898
- WO-A-96/07631
- US-A- 3 725 208
- US-A- 4 600 795

## Description

La présente invention porte sur un perfectionnement du procédé classique de purification de l'acide acrylique, et en particulier des étapes visant à obtenir un monomère de très haute pureté, destiné à la fabrication de polymères techniques à très hautes masses moléculaires.

La principale voie de synthèse de l'acide acrylique utilisée industriellement aujourd'hui met en jeu une oxydation catalytique du propylène, générant intermédiairement l'acroléine. Cette réaction produit également des impuretés secondaires, parmi lesquelles des impuretés carbonylées du type aldéhydes, comme le furfuraldéhyde (ou furfural), le benzaldéhyde, en plus de l'acroléine. Ces composés sont très gênants, même à des teneurs extrêmement faibles, car ils rendent impossible la fabrication de polymères de hautes masses moléculaires, recherchés dans de nombreuses applications.

La présente invention concerne donc un procédé de purification de l'acide acrylique, et plus particulièrement une étape de ce procédé dans laquelle on élimine les impuretés carbonylées présentes dans le flux général (aldéhydes et cétones), par addition d'un composé de type hydrazine pendant une étape de distillation, le composé de type hydrazine formant avec ces impuretés des composés lourds, qui sont purgés en pied de colonne de distillation.

Les procédés classiques de purification de l'acide acrylique mettent en jeu des étapes de distillation successives visant à éliminer les impuretés légères (eau, acroléine, acide acétique, ...) et les impuretés lourdes (acide maléique, acide acryloxypropionique, ...), qui sont formées pendant les étapes de réaction ou de purification.

Au cours de ces étapes de purification par distillation, il arrive fréquemment que des polymères soient formés, sous l'effet thermique, et ceci même dans des conditions opératoires visant, par exemple en conduisant les distillations sous pression réduite, à réduire la température des flux riches en monomères polymérisables. Dans le cas de l'acide acrylique, le polymère étant insoluble dans le monomère, il précipite dans le milieu et occasionne des dépôts sur certaines parties de l'installation, comme les échangeurs.

La formation de ces dépôts solides est particulièrement gênante, car ceux-ci constituent une couche isolante réduisant l'échange thermique, qui a pour conséquence de provoquer l'augmentation de la chauffe pour maintenir une température constante dans le bouilleur, ce qui entraîne l'aggravation du phénomène de polymérisation. On est alors contraint d'arrêter rapidement la distillation pour opérer une nettoyage difficile et coûteux de l'installation.

Il est bien connu que la distillation de monomères acryliques, qui polymérisent facilement sous l'action de radicaux formés par exemple par effet thermique, nécessite de mettre en oeuvre des inhibiteurs de polymérisation, particulièrement pendant les étapes de distillation. Les composés typiquement utilisés dans ce but sont, par exemple, les dérivés phénoliques comme l'hydroquinone ou l'éther méthylique de l'hydroquinone ; la phénothiazine et ses dérivés ; les dérivés de la famille des thiocarbamates ; les composés à groupement nitrosa ; les quinones ; ou encore les amines aromatiques.

Malgré l'utilisation de ces inhibiteurs de polymérisation, des polymères peuvent s'accumuler graduellement, plus ou moins rapidement selon l'étape de purification et les conditions opératoires des équipements traversés par le flux riche en monomère, sous la forme de dépôts gênants.

Ces problèmes apparaissent de manière particulièrement sensible lors d'une étape de purification ultime de l'acide acrylique.

Dans l'objectif d'obtenir une qualité d'acide acrylique de très haute pureté, l'élimination de toutes ces impuretés, jusqu'à des teneurs extrêmement faibles, n'est pas économiquement réalisable par une simple séparation par distillation. En particulier, en raison de leur volatilité proche de celle de l'acide acrylique, les impuretés carbonylées précitées ne peuvent être éliminées efficacement, jusqu'aux teneurs extrêmement faibles qui sont visées, par une seule opération de distillation.

Pour éliminer les impuretés aldéhydiques, le brevet américain US-A-3 725 208 décrit un traitement chimique consistant à ajouter des amines dans le mélange impur, puis à distiller le mélange obtenu. La famille des amines est particulièrement adaptée pour atteindre cet objectif, car ces composés présentent la particularité de former, avec les aldéhydes, des composés lourds, facilement séparables de l'acide acrylique en pied de colonne de distillation. Parmi les réactifs qui présentent la meilleure efficacité, sont décrits, en particulier, ceux qui font partie de la famille des hydrazines, comme la glycine (brevet japonais n° J 7 500 014) ou l'hydrazine elle-même ou ses dérivés (brevet américain US-A-3 725 208 et brevet japonais J 7 430 312) ou encore l'aminoguanidine (brevet européen EP-B-270 999) ou leurs sels.

Les traitements chimiques qui sont décrits présentent tous l'inconvénient de générer de l'eau lors de la réaction de l'aldéhyde avec le réactif aminé. La présence de cette impureté dans l'acide acrylique est également dommageable vis-à-vis de la réactivité du monomère dans ses applications les plus techniques. Pour cette raison, il peut être particulièrement intéressant de réaliser ce traitement chimique pendant une étape de distillation visant à éliminer l'eau et les composés légers en tête, avant l'étape de distillation de l'acide acrylique destinée à séparer les composés lourds, comme il est décrit dans le brevet japonais J 7 495 920.

Un deuxième inconvénient majeur des traitements chimiques d'élimination des aldéhydes par les amines est qu'ils entraînent une réduction significative de la stabilisation du milieu. La fonction amine de ces composés leur confère la propriété de réagir non seulement avec les aldéhydes, mais aussi avec l'acide acrylique lui-même, pour former des sels par réaction sur la partie carboxylique de la molécule, ou des composés d'addition de Michael par réaction de l'amine sur la double liaison acrylique.

Les produits de réaction des amines sur l'acide acrylique entraînent une sensibilité exacerbée du milieu réactionnel vis-à-vis de la polymérisation. Malgré l'utilisation d'inhibiteurs classiquement mis en oeuvre pour la distillation de ce monomère, lorsque ce traitement est réalisé de manière spécifique dans cette distillation, en vue d'éliminer en pied les composés d'addition lourds des aldéhydes avec l'amine, on observe des dépôts de polymères, particulièrement au niveau de la paroi chaude du bouilleur.

La formation de ces dépôts solides entraîne rapidement des problèmes de bouchages de tuyauteries ou de modification des échanges thermiques décrits plus haut, qui nécessitent un arrêt de l'installation pour nettoyage.

Pour réduire ces effets néfastes, la demande de brevet européen EP-A1-0 648 732 revendique l'utilisation d'un acide sulfonique organique au cours du traitement d'élimination des aldéhydes par une amine du type hydrazine ou aminoguanidine. Cette amélioration présente plusieurs inconvénients majeurs. En premier lieu, les acides sulfoniques décrits sont corrosifs et nécessitent d'utiliser des matériaux coûteux pour les parties de l'installation en contact avec ceux-ci. Par ailleurs, les quantités d'additifs mises en jeu sont importantes, puisqu'ils doivent être utilisés en rapport molaire excédentaire vis-à-vis du composé aminé, ce qui rend le traitement coûteux.

Dans le même objectif d'éviter les dépôts de polymères dans cette étape du procédé de purification de l'acide acrylique, la demande de brevet britannique GB-A-2 285 046 décrit une amélioration consistant à réaliser le traitement d'élimination des impuretés par les hydrazines, lors d'une distillation, en ajoutant un composé dithiocarbamate de cuivre. Les composés de cette famille des thiocarbamates métalliques sont bien connus en tant qu'inhibiteurs de polymérisation de l'acide acrylique et des autres monomères acryliques et méthacryliques. Malheureusement, ils présentent l'inconvénient d'entraîner, dans les sous-produits lourds non distillables de l'atelier, des résidus métalliques qui les rendent difficiles à éliminer. Ceux-ci peuvent en effet provoquer l'encrassement des fours d'incinération, nécessitant un coût d'élimination élevé.

De manière générale, l'addition d'inhibiteurs de polymérisation ne suffit pas à éliminer totalement la formation de polymères et matières solides pendant le traitement chimique d'élimination des impuretés carbonylées de l'acide acrylique.

La Société déposante a eu l'idée nouvelle de réduire les inconvénients dus aux dépôts de solides pendant les étapes de purification de l'acide acrylique, et en particulier pendant cette étape critique de distillation en présence de dérivés d'hydrazines, en empêchant leur accrochage aux parois sensibles de l'outil de distillation, grâce à l'utilisation d'agents de surface, encore appelés surfactants. Ces produits ont la particularité commune de présenter dans leur structure une partie hydrophile et une partie hydrophobe. Cette structure leur confère des propriétés, principalement mise à profit dans les milieux aqueux. Citons par exemple la détergence, dans laquelle la molécule est utilisée pour faciliter l'élimination de souillures et salissures dans l'eau, la dispersion, pour augmenter la stabilité de la suspension de petites particules solides au sein d'un liquide aqueux, la propriété d'émulsionnant, lorsque l'agent de surface facilite la dispersion, sous forme de fines gouttelettes, d'un liquide hydrophobe dans l'eau ou au contraire d'eau dans un liquide hydrophobe, la propriété de moussants ou au contraire d'antimousses lorsque le composé provoque ou empêche la formation de mousses, ou encore la solubilisation, lorsque le produit est utilisé pour augmenter la solubilité apparente dans l'eau des corps peu solubles.

Un grand nombre de produits présentant une propriété de ce type sont connus. Ils sont communément classifiés, selon leur structure, en agents de surface anioniques, cationiques, amphotères et non-ioniques.

De manière surprenante, la Société déposante a maintenant découvert que l'addition de très faibles quantités de composés de la classe des agents de surface non-ioniques, et plus particulièrement à l'intérieur de cette classe de la famille des éthers de polysaccharides, seuls ou en combinaison, en présence d'inhibiteurs de polymérisation, réduit significativement la quantité de dépôts de polymères, particulièrement dans les bouilleurs des colonnes de distillation de l'acide acrylique, et principalement dans les échangeurs situés en pied ou à l'alimentation de colonne, lors d'une étape de distillation de l'acide acrylique réalisée dans un milieu essentiellement anhydre. Cette amélioration est particulièrement mise à profit lorsque la distillation de l'acide acrylique est réalisée en présence de composés du type hydrazine, en vue d'en éliminer les impuretés aldéhydiques. Ainsi, conformément au procédé de la présente invention, on augmente significativement la durée de distillation en réduisant fortement les encrassements causés par les dépôts. En outre, lorsque l'invention est mise en oeuvre dans le cadre d'un procédé de purification utilisant un composé aminé visant à éliminer les impuretés aldéhydiques de l'acide acrylique, les faibles dépôts, qui sont accumulés au bout d'un temps beaucoup plus long que dans les conditions connues dans l'art antérieur, sont beaucoup plus facilement éliminables que ceux de cet art antérieur, puisqu'un simple lavage à l'eau permet de nettoyer efficacement les équipements encrassés.

La présente invention a donc pour objet un procédé de purification de l'acide acrylique visant à éliminer les impuretés du type polymère dans les endroits des colonnes à distiller où elles ont tendance à s'accumuler, et plus particulièrement un procédé visant à éliminer les impuretés polymériques qui sont formées pendant une étape d'élimination des impuretés aldéhydiques de l'acide acrylique, étape suivant laquelle on conduit une distillation du milieu contenant l'acide acrylique à purifier en ajoutant dans ledit milieu au moins un composé aminé de type hydrazine et en purgeant, en pied de colonne de distillation, des composés lourds formés par ledit ou lesdits composés aminés de type hydrazine avec lesdites impuretés, caractérisé par le fait qu'on conduit la distillation de l'acide acrylique à purifier également en présence d'au moins un composé de la classe des agents de surface non-ioniques et d'au moins un inhibiteur de polymérisation.

Les agents de surface non-ioniques utiles conformément à la présente sont notamment :
. les composés de la famille des éthers et acétates de polysaccharides, de préférence les éthers, et en particulier ceux qui sont dérivés de la cellulose ou de l'amidon tels que ceux représentés par la formule (I) : 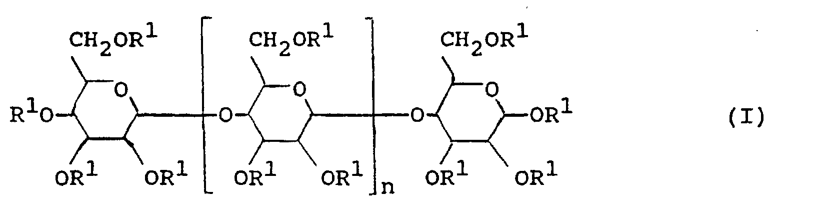 dans laquelle :
   - les R¹ représentent indépendamment H ; un groupement alkyle en C₁-C₄ ; CH₃CO- ; 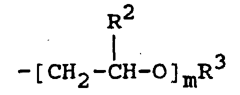 avec R² = H, CH₃ ou C₂H₅,
      m= entier de 1 à 20 et R³ = H ou alkyle en C₁-C₄ ou CH₃CO- ; et
   - n est un entier supérieur à 1, représentant le nombre de maillons d'une chaîne polymérique ;
. les dérivés d'éthylène et de propylène glycols ou de leurs éthers, représentés par la formule (II) : 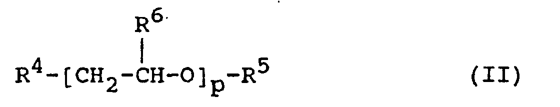 dans laquelle :
   - R⁴ et R⁵ représentent chacun indépendamment HO- ou R⁷O- ou R⁷-C₆H₄O- avec R⁷ représentant un groupement alkyle en C₁-C₂₀ ;
   - R⁶ représente H ou CH₃ ; et
   - p est un entier de 3 à 20 ;
. les dérivés d'esters de glycols, représentés par la formule (III) : 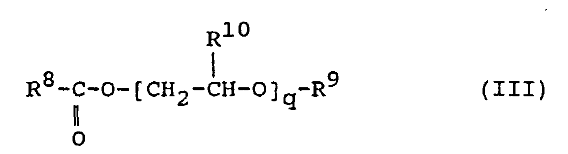 dans laquelle :
   - R⁸ représente un groupement alkyle en C₈-C₂₀ ;
   - R⁹ représente H ou un groupement alkyle en C₈-C₂₀ ;
   - R¹⁰ représente H ou CH₃ ;
   - q est un entier de 1 à 20 ;
. les dérivés d'esters de glycérol, représentés par la formule (IV) : 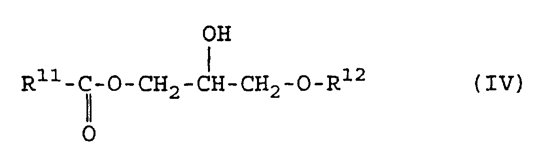 dans laquelle :
   - R¹¹ représente un groupement alkyle en C₈-C₂₀ ; et
   - R¹² représente H ou 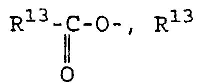 représentant un groupement alkyle en C₈-C₂₀ ;
. les dérivés d'amides carboxyliques, représentés par la formule (V) : 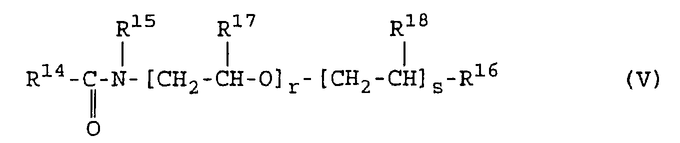 dans laquelle :
   - R¹⁴ représente un groupement alkyle en C₈-C₂₀ ;
   - R¹⁵ représente H ou 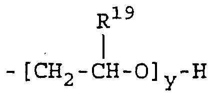 avec R¹⁹ représentant H ou CH₃ et y étant un entier de 1 à 5 ;
   - R¹⁶ représente H ou 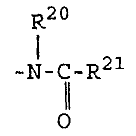 où R²⁰ représente H ou 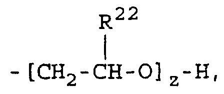 avec R²² représentant H ou CH₃ et z est un entier de 1 à 5, et R²¹ est un alkyle en C₈-C₂₀ ;
   - R¹⁷ et R¹⁸ représentant chacun indépendamment H ou CH₃ ;
   - r valant 0 ou étant un entier de 1 à 20 ; et
   - s valant 0 ou 1.

On utilise de préférence les composés de formule (I) seuls ou en mélange avec les agents de surface décrits des formules (II) à (V) ci-dessus.

Le ou les composés agents de surface non-ioniques peuvent être envoyés dans le flux contenant l'acide acrylique à purifier seuls (lorsqu'il s'agit de produits liquides) ou en solution ou suspension dans un solvant. De manière préférentielle, lorsqu'il s'agit de composés solides, ceux-ci sont d'abord mis en solution ou en suspension dans un solvant, comme, par exemple, l'acide acrylique, l'acide acétique, l'acide propionique, l'acide maléique, l'anhydride maléique et l'eau. De manière encore plus avantageuse, les composés agents de surface non-ioniques solides sont ajoutés en solution dans un milieu contenant de l'acide acrylique, comme la solution riche en inhibiteurs de polymérisation qui est utilisée pour injecter ces derniers en tête ou dans l'alimentation des colonnes de distillation.

Généralement, on ajoute le ou les composés agents de surface non-ioniques à raison de 10 - 10 000 ppm, de préférence 10 - 1000 ppm par rapport au milieu contenant l'acide acrylique à purifier.

On ajoute également au milieu contenant l'acide acrylique à purifier à raison notamment de 5 - 10 000 ppm, de préférence de 10 - 5 000 ppm, par rapport au milieu contenant l'acide acrylique à purifier, au moins un inhibiteur de polymérisation, choisi notamment parmi les dérivés phénoliques comme l'hydroquinone et ses dérivés, tels que l'éther méthylique de l'hydroquinone ; la phénothiazine et ses dérivés, tels que le bleu de méthylène; les thiocarbamates métalliques, tels que le dibutyldithiocarbamate de cuivre ; les composés à groupements nitroso, tels que la N-nitroso phényl hydroxylamine ; les quinones telles que la benzoquinone ; les dérivés de paraphénylènediamine, représentés par la formule générale (VI) : 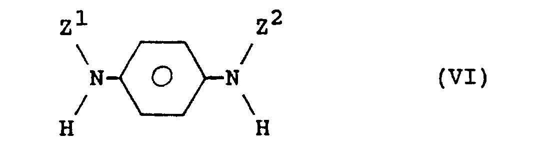 dans laquelle Z¹ et Z² représentent chacun indépendamment un radical alkyle, aryle, alkylaryle ou arylalkyle.

Dans le cadre d'un procédé mettant en jeu un composé aminé de type hydrazine, visant à éliminer les impuretés aldéhydiques présentes dans l'acide acrylique, on choisit le composé aminé de type hydrazine parmi l'hydrazine ; l'hydrate d'hydrazine ; les alkylhydrazines telles que la cyclohexylhydrazine, l'hexadécylhydrazine ; la phénylhydrazine ; la 2-naphtylhydrazine ; la tolylhydrazine ; la p-nitrophénylhydrazine ; la 2,4-dinitrophénylhydrazine ; la glycine ; la guanidine ; l'aminoguanidine ; et leurs sels. Le ou les composés aminés de type hydrazine sont généralement ajoutés à raison de 10-10 000 ppm, de préférence de 100 - 5 000 ppm, par rapport au milieu contenant l'acide acrylique à purifier.

Il s'est par ailleurs avéré que, conformément à la présente invention, on peut avantageusement ajouter le ou les composés aminés de type hydrazine dans une quantité telle que le rapport molaire composé(s) de type hydrazine/somme des aldéhydes présents dans l'alimentation soit de 0,5/1 - 10/1, en particulier de 1/1-5/1.

Le procédé selon la présente invention peut être conduit en mode discontinu, ou encore en mode continu, par exemple dans le flux d'acide acrylique à purifier alimentant la colonne de distillation du monomère.

De façon encore plus avantageuse, on peut réaliser le traitement, qui génère de l'eau lors de la réaction des composés de type hydrazine avec les impuretés carbonylées, dans le flux alimentant une colonne permettant d'éliminer les impuretés légères et l'eau en tête, le flux de pied de cette colonne d'étêtage étant ensuite envoyé en alimentation de l'ultime colonne réalisant la distillation de l'acide acrylique pur en tête et l'élimination des composés lourds en pied (composés de réaction des impuretés aldéhydiques avec les composants de type hydrazine, inhibiteurs, agents de surface non-ioniques, ...). C'est ainsi que l'on peut conduire la distillation en deux étapes successives :
- la première étape, dans une première colonne de d'étêtage (C1), alimentée par le flux d'acide acrylique à purifier et recevant le ou les composés aminés de type hydrazine, pour éliminer, en tête, les impuretés légères et l'eau générée lors de la réaction du ou des composés de type hydrazine avec les impuretés aldéhydiques ; et
- la seconde étape, dans une seconde colonne de distillation (C2), alimentée par le flux de pied de ladite colonne d'étêtage, pour réaliser, en tête, la distillation de l'acide acrylique pur, et, en pied, l'élimination des composés lourds, consistant notamment en les produits de réaction des impuretés aldéhydiques avec les composés de type hydrazine, le ou les inhibiteurs de polymérisation et le ou les agents de surface non-ioniques,
le ou les inhibiteurs de polymérisation étant ajoutés en tête de la colonne (C1) et en tête de la colonne (C2), et éventuellement dans l'alimentation de ces deux colonnes,
le ou les composés agents de surface non-ioniques étant ajoutés seuls ou mélangés dans l'alimentation de la colonne (C1) et/ou en tête des colonnes (C1) et (C2), auquel cas ils peuvent être injectés par exemple dans une solution à base de solvant ou d'acide acrylique contenant les inhibiteurs, qui est utilisée pour envoyer ces inhibiteurs en tête desdites colonnes.

La présente invention va maintenant être décrite à l'aide des Exemples et Exemples comparatifs suivants.

### EXEMPLES

Dans les exemples suivants, les pourcentages sont exprimés en poids rapportés à la masse d'acide acrylique à purifier, et les abréviations suivantes sont utilisées :

### Inhibiteurs :

- • PTZ :: phénothiazine
- • HQ :: hydroquinone
- • CB :: di-n-butyldithiocarbamate de cuivre
- • DSB-PPDA :: N,N'-di-sec.butyl paraphénylènediamine

### Agents de surface :

- • NP10 :: nonylphénol polyéthoxylé (10 éthoxy) - produit commercialisé sous la dénomination "Tergipol NP10" (agent de surface non-ionique)
- • Rewopal MT65 :: polyglycoléther à base de coprah - dénomination commerciale d'un agent de surface non-ionique
- • DBSS :: dodécylbenzène sulfonate de sodium (agent de surface anionique)
- • DMDNO :: N,N-diméthyldodécylamine,N-oxyde (détergent cationique)
- • HPC KLUCEL H :: hydroxypropylcellulose - dénomination commerciale d'un agent de surface non-ionique
- • MHPC xxxxx :: composés de la famille des méthyl-hydroxypropylcelluloses, caractérisés par la viscosité de leur solution à 2% dans l'eau à 20°C, qui est indiquée par l'extension "xxxxx" (agents de surface non-ioniques)
- • MHEC xxxxx :: composés de la famille des méthyl-hydroxyéthylcelluloses, caractérisés par la viscosité de leur solution à 2% dans l'eau à 20°C, qui est indiquée par l'extension "xxxxx" (agents de surface non-ioniques).

### Mode opératoire général des exemples 1 et 2 (références), 3 et 4 (comparatifs) et 5 à 13 (de l'invention) :

On utilise un équipement de distillation en verre, fonctionnant en mode continu, qui comprend :
- une colonne à distiller C1, fonctionnant sous une pression réduite de 1,07 x 10⁴ Pa (80 mm Hg), comprenant des plateaux perforés représentant une efficacité de 6 plateaux théoriques, équipée :
   - en pied, d'un bouilleur à recirculation forcée à travers une pompe, chauffé par circulation d'huile thermostatée dans la double enveloppe, comprenant une injection d'air ;
   - en tête, d'un condenseur, d'un pot de reflux, d'une pompe et d'un système de mesure permettant de renvoyer une partie du distillat en reflux en tête de colonne ;
   - d'une pompe envoyant une solution d'inhibiteur(s) dissous dans l'acide acrylique dans le circuit de reflux de la colonne ; et
   - d'une alimentation située aux 2/3 de la colonne, préalablement préchauffée par un échangeur à double enveloppe, chauffé à l'huile, et qui reçoit avant l'échangeur un débit connu d'hydrate d'hydrazine ;
- une colonne à distiller C2, de type Vigreux, fonctionnant sous une pression réduite de 1,07 x 10⁴ Pa (80 mm Hg), et présentant une efficacité de 6 plateaux théoriques, équipée :
   - en pied, d'un bouilleur à recirculation forcée à travers une pompe, chauffé par circulation d'huile thermostatée dans la double enveloppe, comprenant une injection d'air ;
   - en tête, d'un condenseur, d'un pot de reflux, d'une pompe et d'un système de mesure permettant de renvoyer une partie du distillat en reflux en tête de colonne ;
   - d'une pompe envoyant une solution d'inhibiteur(s) dissous dans l'acide acrylique dans le circuit de reflux de la colonne ; et
   - d'une alimentation située dans le bouilleur de la colonne.

La solution d'inhibiteur(s) dissous dans l'acide acrylique, envoyée en tête de colonne C1 contient également, excepté pour les exemples 1 et 2 de référence, les agents de surface.

Dans la colonne C1, on distille 5% du flux alimentant cette colonne. Dans la colonne C2, 90% du flux d'alimentation sont distillés en tête. Les températures sont de 83°C en pied de colonne C1 et de 86°C en pied de colonne C2. Les deux colonnes fonctionnent en série, le flux de pied de colonne C1 étant envoyé en continu en pied de la colonne C2.

Le flux d'alimentation de la colonne C1 est constitué d'acide acrylique contenant des impuretés aldéhydiques à hauteur de 150-200 ppm d'acroléine, 190-250 ppm de furfural et 50-100 ppm de benzaldéhyde, par rapport à ce flux.

La durée des essais est de 12 heures, au terme de laquelle on vide l'équipement de son flux liquide, on remplit le bouilleur situé au pied de la colonne C1 par de l'eau distillée, on fait circuler cette eau à température ambiante, pendant 1 heure, à l'aide de la pompe assurant la recirculation dans le bouilleur pendant l'essai. On s'assure qu'après ce lavage, il ne subsiste aucun résidu solide sur la surface du bouilleur. L'eau de lavage est récupérée dans un ballon et on l'évapore à sec. Le résidu obtenu après évaporation complète est pesé. Il permet de quantifier l'importance du phénomène d'encrassement selon les conditions opératoires des différents essais.

### Exemples 1 et 2 (de référence) :

Ces exemples décrivent des distillations effectuées avec traitement d'élimination des aldéhydes par l'hydrate d'hydrazine, en présence d'inhibiteurs "classiques", sans ajout d'agents de surface.

### Exemples 3 à 14 :

Ces exemples décrivent des distillations effectuées dans des conditions identiques à celles des exemples 1 et 2 de référence, excepté le fait qu'on ajoute, dans la solution d'inhibiteurs dans l'acide acrylique envoyée en tête de colonne C1, un ou plusieurs agent(s) de surface.

### Exemples 3 et 4 :

Les agents de surface utilisés appartiennent respectivement au groupe des agents de surface anioniques et cationiques. L'encrassement du bouilleur de colonne C1 est supérieur ou comparable à celui des exemples de référence 1 et 2.

### Exemples 5 à 14 :

Les agents de surface testés appartiennent au groupe des agents de surface non-ioniques. Les résultats d'encrassement au niveau du bouilleur de la colonne C1 sont sensiblement améliorés par rapport aux exemples de référence et comparatifs 1 à 4.

Les agents de surface à base d'éther de cellulose (exemples 7 à 14) réduisent les encrassements de façon particulièrement nette.

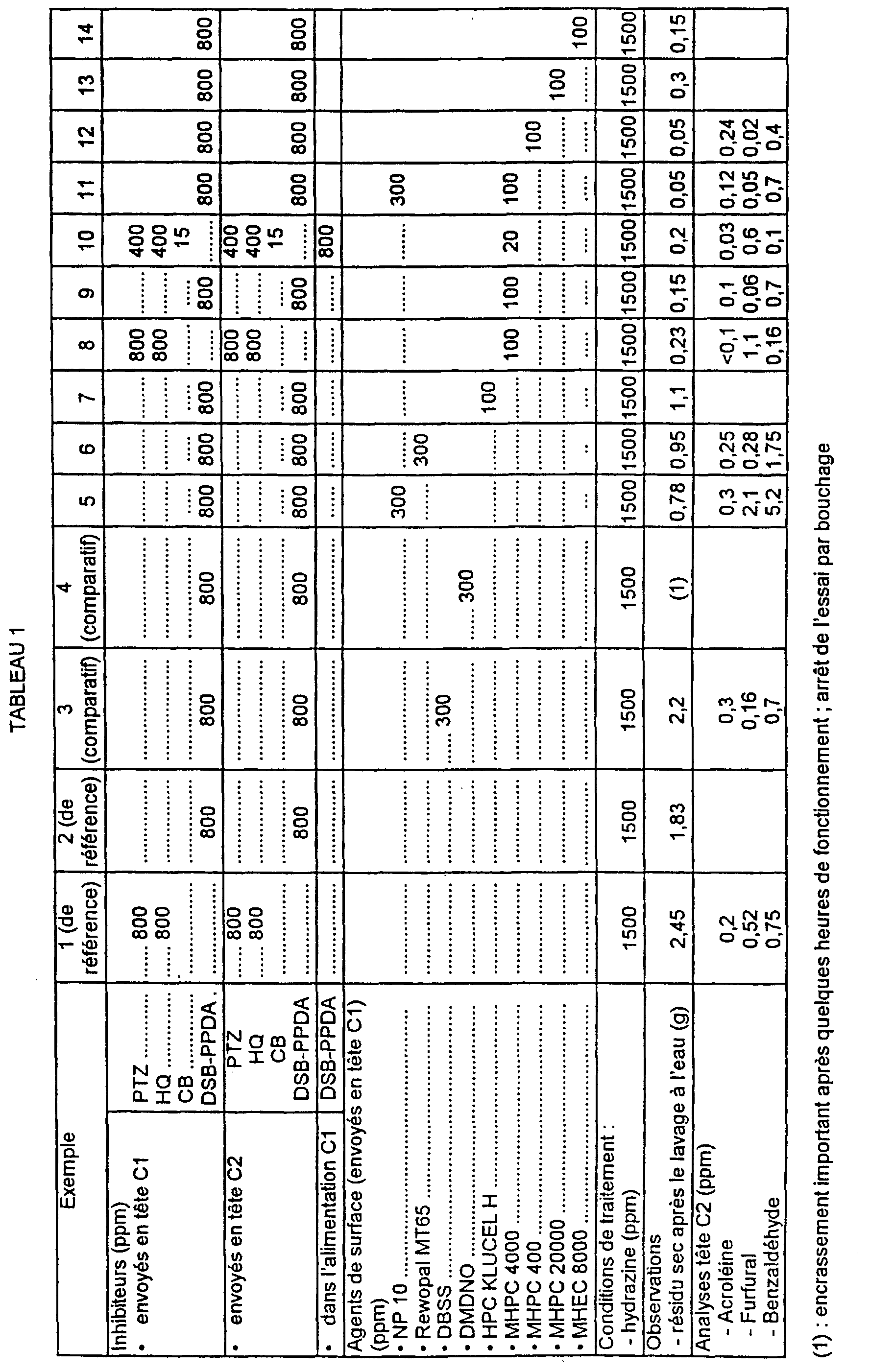

## Revendications

1. Procédé de purification de l'acide acrylique visant à éliminer les impuretés du type polymère dans les endroits des colonnes à distiller où elles ont tendance à s'accumuler, en particulier visant à éliminer les impuretés polymériques qui sont formées pendant une étape d'élimination des impuretés aldéhydiques de l'acide acrylique, étape suivant laquelle on conduit une distillation du milieu contenant l'acide acrylique à purifier en ajoutant dans ledit milieu au moins un composé aminé de type hydrazine et au moins un inhibiteur de polymérisation et en purgeant, en pied de colonne de distillation, des composés lourds formés par ledit ou lesdits composés aminés de type hydrazine avec lesdites impuretés, **caractérisé par le fait qu'**on conduit la distillation de l'acide acrylique à purifier également en présence d'au moins un composé de la classe des agents de surface non-ioniques.

2. Procédé selon la revendication 1, **caractérisé par le fait qu'**on choisit les agents de surface non-ioniques parmi :
• les composés de la famille des éthers et acétates de polysaccharides, de préférence les éthers, et en particulier ceux qui sont dérivés de la cellulose ou de l'amidon tels que ceux représentés par la formule (I) 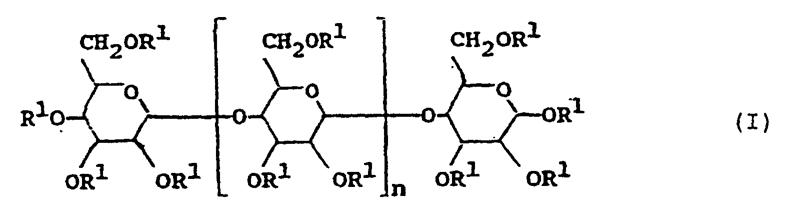 dans laquelle :
- les R¹ représentent indépendamment H ; un groupement alkyle en C₁-C₄ ; CH₃CO- ; 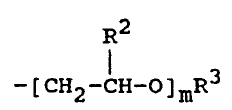 avec R² = H, CH₃ ou C₂H₅, m = entier de 1 à 20 et R³ = H ou alkyle en C₁-C₄ ou CH₃CO- ; et
- n est un entier supérieur à 1, représentant le nombre de maillons d'une chaîne polymérique ;
• les dérivés d'éthylène et de propylène glycols ou de leurs éthers, représentés par la formule (II) : 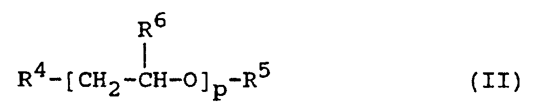 dans laquelle :
- R⁴ et R⁵ représentent chacun indépendamment HO- ou R70- ou R⁷-C₆H₄O- avec R⁷ représentant un groupement alkyle en C₁-C₂₀ ;
- R⁶ représente H ou CH₃ ; et
- p est un entier de 3 à 20 ;
• les dérivés d'esters de glycols, représentés par la formule (III) : 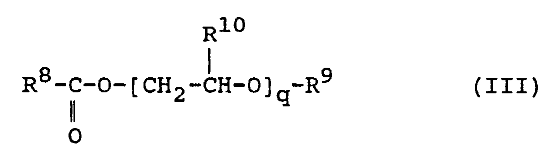 dans laquelle :
- R8 représente un groupement alkyle en C₈-C₂₀ ;
- R⁹ représente H ou un groupement alkyle en C₈-C₂₀ ;
- R¹⁰ représente H ou CH₃ ;
- q est un entier de 1 à 20 ;
• les dérivés d'esters de glycérol, représentés par la formule (IV) : 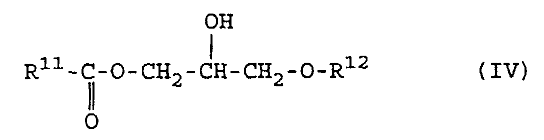 dans laquelle :
- R¹¹ représente un groupement alkyle en C₈-C₂₀ ; et
- R¹² représente H ou 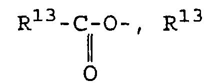 représentant un groupement alkyle en C₈-C₂₀ ;
• les dérivés d'amides carboxyliques, représentés par la formule (V) : 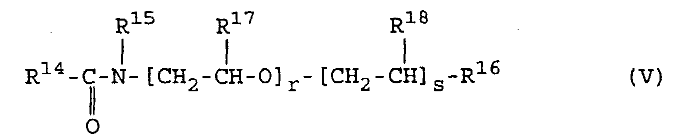 dans laquelle :
- R¹⁴ représente un groupement alkyle en C₈-C₂₀ ;
- R¹⁵ représente H ou 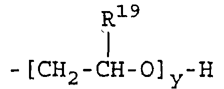 avec R¹⁹ représentant H ou CH₃ et y étant un entier de 1 à 5 ;
- R¹⁶ représente H ou 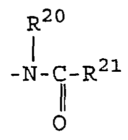 où R²⁰ représente H ou 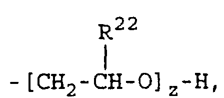 avec R²² représentant H ou CH₃ et z est un entier de 1 à 5, et R²¹ est un alkyle en C₈-C₂₀ ;
- R¹⁷ et R¹⁸ représentant chacun indépendamment H ou CH₃ ;
- r valant 0 ou étant un entier de 1 à 20 ; et
- s valant 0 ou 1.

3. Procédé selon l'une des revendications 1 et 2, **caractérisé par le fait que** l'on envoie le ou les agents de surface non-ioniques dans le flux contenant l'acide acrylique seuls ou en solution ou suspension dans un solvant, tel que l'acide acrylique, l'acide acétique, l'acide propionique, l'acide maléique, l'anhydride maléique et l'eau, ou en solution dans un milieu contenant de l'acide acrylique, comme la solution riche en inhibiteurs de polymérisation qui est utilisée pour injecter ces derniers en tête ou dans l'alimentation des colonnes de distillation.

4. Procédé selon l'une des revendications 1 à 3, **caractérisé par le fait que** l'on ajoute le ou les agents de surface non-ioniques à raison de 10 - 10 000 ppm, de préférence 10 - 1000 ppm par rapport au milieu contenant l'acide acrylique à purifier.

5. Procédé selon l'une des revendications 1 à 4, **caractérisé par le fait que** l'on choisit les inhibiteurs de polymérisation parmi les dérivés phénoliques comme l'hydroquinone et ses dérivés, tels que l'éther méthylique de l'hydroquinone ; la phénothiazine et ses dérivés, tels que le bleu de méthylène; les thiocarbamates métalliques, tels que le dibutyldithiocarbamate de cuivre ; les composés à groupements nitroso, tels que la N-nitroso phényl hydroxylamine ; les quinones telles que la benzoquinone ; les dérivés de paraphénylènediamine, représentés par la formule générale (VI) : 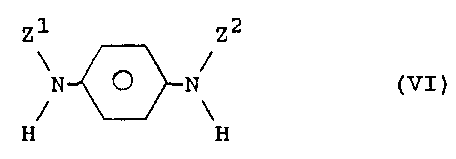 dans laquelle Z¹ et Z² représentent chacun indépendamment un radical alkyle, aryle, alkylaryle ou arylalkyle.

6. Procédé selon l'une des revendications 1 à 5, **caractérisé par** le fait on ajoute le ou les inhibiteurs de polymérisation à raison de 5 - 10 000 ppm par rapport au milieu contenant l'acide acrylique à purifier.

7. Procédé selon l'une des revendications 1 à 6, **caractérisé par le fait qu'**on choisit le composé aminé de type hydrazine parmi l'hydrazine ; l'hydrate d'hydrazine ; les alkylhydrazines telles que la cyclohexylhydrazine, l'hexadécylhydrazine ; la phénylhydrazine ; la 2-naphtylhydrazine ; la tolylhydrazine ; la p-nitrophénylhydrazine ; la 2,4-dinitrophénylhydrazine ; la glycine ; la guanidine ; l'aminoguanidine ; et leurs sels.

8. Procédé selon l'une des revendications 1 à 7, **caractérisé par le fait qu'**on ajoute le ou les composés aminés de type hydrazine à raison de 10 - 10 000 ppm par rapport au milieu contenant l'acide acrylique à purifier.

9. Procédé selon l'une des revendications 1 à 8, **caractérisé par le fait qu'**on ajoute le ou les composés aminés de type hydrazine dans une quantité telle que le rapport molaire composé(s) de type hydrazine/somme des aldéhydes présents dans l'alimentation soit de 0,5/1 - 10/1, en particulier de 1/1 - 5/1.

10. Procédé selon l'une des revendications 1 à 9, **caractérisé par le fait qu'**il est conduit en mode discontinu, ou encore en mode continu, par exemple dans le flux d'acide acrylique à purifier alimentant la colonne de distillation du monomère.

11. Procédé selon l'une des revendications 1 à 10, **caractérisé par le fait qu'**on conduit la distillation en deux étapes successives :
- la première étape, dans une première colonne de d'étêtage (C1), alimentée par le flux d'acide acrylique à purifier et recevant le ou les composés aminés de type hydrazine, pour éliminer, en tête, les impuretés légères et l'eau générée lors de la réaction du ou des composés de type hydrazine avec les impuretés aldéhydiques ; et
- la seconde étape, dans une seconde colonne de distillation (C2), alimentée par le flux de pied de ladite colonne d'étêtage (C1), pour réaliser, en tête, la distillation de l'acide acrylique pur, et, en pied, l'élimination des composés lourds, consistant notamment en les produits de réaction des impuretés aldéhydiques avec les composés de type hydrazine, le ou les inhibiteurs de polymérisation et le ou les agents de surface non-ioniques,
le ou les inhibiteurs de polymérisation étant ajoutés en tête de la colonne (C1) et en tête de la colonne (C2), et éventuellement dans l'alimentation de ces deux colonnes,
le ou les composés agents de surface non-ioniques étant ajoutés seuls ou mélangés dans l'alimentation de la colonne (C1) et/ou en tête des colonnes (C1) et (C2), auquel cas ils peuvent être injectés par exemple dans une solution à base de solvant ou d'acide acrylique contenant les inhibiteurs, qui est utilisée pour envoyer ces inhibiteurs en tête desdites colonnes.

## Patentansprüche

1. Verfahren zur Reinigung von Acrylsäure, mit dem die Verunreinigungen vom Polymertyp an den Stellen der Destillationskolonnen beseitigt werden sollen, an denen sie sich bevorzugt anreichern, und mit dem insbesondere die Polymerverunreinigungen beseitigt werden sollen, die während eines Schrittes zum Entfernen der aldehydischen Verunreinigungen von Acrylsäure gebildet werden, wobei nach diesem Schritt eine Destillation des Mediums durchgeführt wird, das die zu reinigende Acrylsäure enthält, wobei in das Medium mindestens eine aminierte Verbindung vom Hydrazintyp und mindestens ein Polymerisationsinhibitor gegeben wird und wobei die aus der oder den aminierten Verbindungen vom Hydrazintyp mit den Verunreinigungen gebildeten hochsiedenden Verbindungen am Fuß der Destillationskolonne entnommen werden, **dadurch gekennzeichnet, daß** die Destillation der zu reinigenden Acrylsäure in Gegenwart mindestens einer Verbindung aus der Gruppe der nichtionischen grenzflächenaktiven Stoffe durchgeführt wird.

2. Verfahren nach Anspruch 1, **dadurch gekennzeichnet, daß** die nichtionischen grenzflächenaktiven Stoffe ausgewählt sind unter:
• den Verbindungen aus der Gruppe der Ether und Acetate von Polysacchariden und vorzugsweise der Ether, insbesondere den Verbindugen, die von Cellulose oder Stärke abgeleitet sind, beispielsweise den Verbindungen der Formel (I): 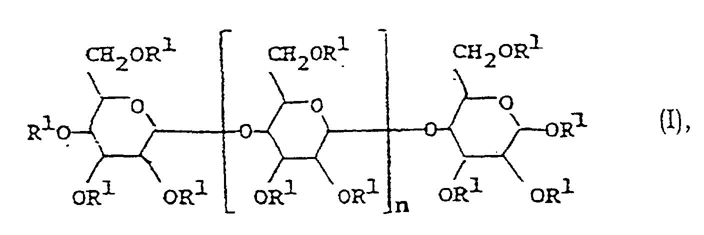 worin bedeuten:
- die Gruppen R¹ unabhängig voneinander H; C₁₋₄-Alkyl; CH₃CO-; 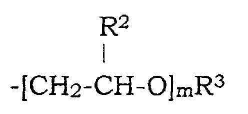 mit R² = H, CH₃ oder C₂H₅,
m = eine ganze Zahl von 1 bis 20 und
R³ = H oder C₁₋₄-Alkyl oder CH₃CO-; und
- n eine ganze Zahl über 1, wobei n die Anzahl der Kettenglieder einer Polymerkette bedeutet;
• den Derivaten von Ethylenglykolen und Propylenglykolen oder deren Ethern der Formel (II): 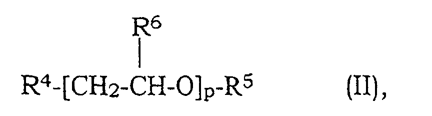 worin bedeuten:
- R⁴ und R⁵ unabhängig voneinander OH- oder R70- oder R⁷-C₆H₄O-, wobei R⁷ eine C₁₋₂₀-Alkylgruppe bedeutet;
- R⁶ H oder CH₃; und
- p eine ganze Zahl von 3 bis 20;
• den Derivaten von Glykolestern der Formel (III): 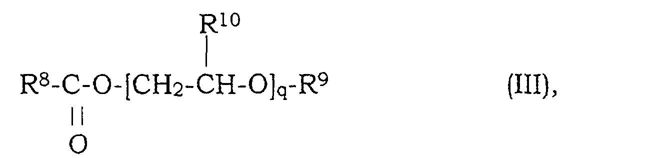 worin bedeuten:
- R⁸ eine C₈₋₂₀-Alkylgruppe;
- R⁹ H oder eine C₈₋₂₀-Alkylgruppe;
- R¹⁰ H oder CH₃;
- q eine ganze Zahl von 1 bis 20;
• den Derivaten von Glycerinestern der Formel (IV): 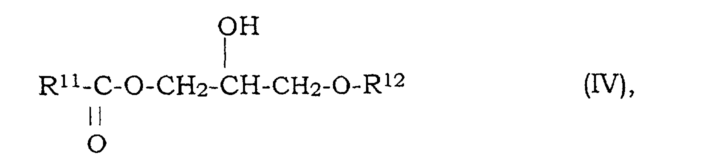 worin bedeuten:
- R¹¹ eine C₈₋₂₀-Alkylgruppe; und 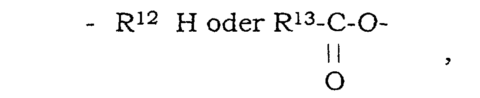 wobei R¹³ eine C₈₋₂₀-Alkylgruppe bedeutet;
• den Derivaten von Carbonsäureamiden der Formel (V): 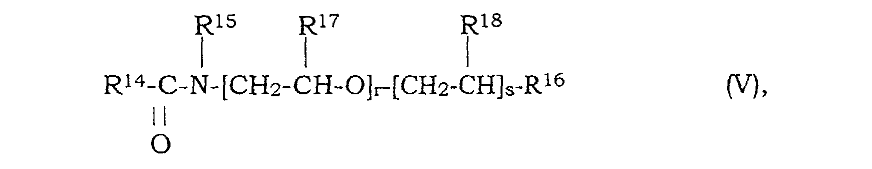 worin bedeuten:
- R¹⁴ eine C₈₋₂₀-Alkylgruppe;
- R¹⁵ H oder 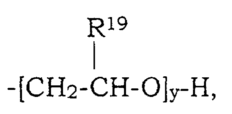 wobei
R¹⁹ H oder CH₃ und
y eine ganze Zahl von 1 bis 5 bedeutet;
- R¹⁶ H oder 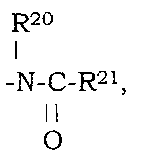 wobei R²⁰H oder 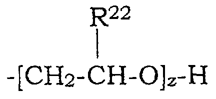 bedeutet,
worin
R²² H oder CH₃ und
z eine ganze Zahl von 1 bis 5 bedeutet, und
R²¹ eine C₈₋₂₀-Alkylgruppe bedeutet;
- R¹⁷ und R¹⁸ unabhängig voneinander H oder CH₃;
- r 0 oder eine ganze Zahl von 1 bis 20; und
- s 0 oder 1.

3. Verfahren nach einem der Ansprüche 1 und 2, **dadurch gekennzeichnet, daß** der nichtionische grenzflächenaktive Stoff oder die nichtionischen grenzflächenaktiven Stoffe in den Strom, der die Acrylsäure enthält, als solche oder in Lösung oder Suspension in einem Lösungsmittel, wie Acrylsäure, Essigsäure, Propionsäure, Maleinsäure, Maleinsäureanhydrid und Wasser, oder in Lösung in einem Medium, das die Acrylsäure enthält, zugeführt werden, beispielsweise in der Lösung mit einem hohen Gehalt an Polymerisationsinhibitoren, die verwendet wird, um diese am Kopf oder in die Zuführung der Destillationskolonnen einzuführen.

4. Verfahren nach einem der Ansprüche 1 bis 3, **dadurch gekennzeichnet, daß** der (die) nichtionische(n) grenzflächenaktive(n) Stoff(e) in Mengenanteilen von 10 bis 10 000 ppm und vorzugsweise 10 bis 1 000 ppm, bezogen auf das Medium, das die zu reinigende Acrylsäure enthält, zugegeben werden.

5. Verfahren nach einem der Ansprüche 1 bis 4, **dadurch gekennzeichnet, daß** die Polymerisationsinhibitoren unter den Phenolderivaten, wie Hydrochinon und seinen Derivaten, beispielsweise dem Hydrochinonmethylether; Phenothiazin und seinen Derivaten, beispielsweise Methylenblau; Metallthiocarbamaten, beispielsweise Kupferdibutyldithiocarbamat; Verbindungen mit Nitrosogruppen, wie beispielsweise N-Nitrosophenylhydroxylamin; Chinonen, wie Benzochinon; und p-Phenylendiaminderivaten der allgemeinen Formel (VI): 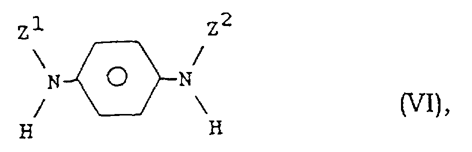 worin Z¹ und Z² unabhängig voneinander Alkyl, Aryl, Alkylaryl oder Arylalkyl bedeuten, ausgewählt sind.

6. Verfahren nach einem der Ansprüche 1 bis 5, **dadurch gekennzeichnet, daß** der Polymerisationsinhibitor oder die Polymerisationsinhibitoren in Mengenanteilen von 5 bis 10 000 ppm, bezogen auf das Medium, das die zu reinigende Acrylsäure enthält, zugegeben werden.

7. Verfahren nach einem der Ansprüche 1 bis 6, **dadurch gekennzeichnet, daß** die aminierte Verbindung vom Hydrazintyp unter Hydrazin; Hydrazinhydrat; Alkylhydrazinen, wie Cyclohexylhydrazin, Hexadecylhydrazin; Phenylhydrazin; 2-Naphthylhydrazin; Tolylhydrazin; p-Nitrophenylhydrazin; 2,4-Dinitrophenylhydrazin; Glycin; Guanidin; Aminoguanidin; und deren Salzen ausgewählt ist.

8. Verfahren nach einem der Ansprüche 1 bis 7, **dadurch gekennzeichnet, daß** die aminierte(n) Verbindung(en) vom Hydrazintyp in Mengenanteilen von 10 bis 10 000 ppm, bezogen auf das Medium, das die zu reinigende Acrylsäure enthält, zugegeben werden.

9. Verfahren nach einem der Ansprüche 1 bis 8, **dadurch gekennzeichnet, daß** die aminierte(n) Verbindung(en) vom Hydrazintyp in einem solchen Mengenanteil zugegeben werden, daß das Molverhältnis der Verbindung(en) vom Hydrazintyp/Summe der in der Zuführung vorliegenden Aldehyde im Bereich von 0,5/1 bis 10/1 und insbesondere 1/1 bis 5/1 liegt.

10. Verfahren nach einem der Ansprüche 1 bis 9, **dadurch gekennzeichnet, daß** es diskontinuierlich oder kontinuierlich durchgeführt wird, beispielsweise in dem Strom der zu reinigenden Acrylsäure, die die Destillationskolonne des Monomers speist.

11. Verfahren nach einem der Ansprüche 1 bis 10, **dadurch gekennzeichnet, daß** die Destillation in zwei aufeinanderfolgenden Schritten durchgeführt wird:
- dem ersten Schritt in einer ersten Kolonne für das Kopfprodukt (C1), die von dem Strom der zu reinigenden Acrylsäure gespeist wird und in die die aminierte(n) Verbindung(en) vom Hydrazintyp gegeben werden, um am Kopf die niedrigsiedenden Verunreinigungen und das Wasser zu entfernen, das bei der Reaktion der Verbindung(en) vom Hydrazintyp mit den aldehydischen Verunreinigungen gebildet wird; und
- dem zweiten Schritt in einer zweiten Destillationskolonne (C2), die mit dem am Fuß der Kolonne für das Kopfprodukt (C1) abgenommenen Strom gespeist wird, um am Kopf die reine Acrylsäure abzudestillieren und am Fuß die hochsiedenden Verbindungen zu entfernen, die insbesondere aus den Produkten der Reaktion von aldehydischen Verunreinigungen mit den Verbindungen vom Hydrazintyp, dem Polymerisationsinhibitor oder den Polymerisationsinhibitoren und dem oder den nichtionischen grenzflächenaktiven Stoffen bestehen,
wobei der Polymerisationsinhibitor oder die Polymerisationsinhibitoren am Kopf der Kolonne (C1) und am Kopf der Kolonne (C2) zugegeben und gegebenenfalls in die Zuführung der beiden Kolonnen gegeben wird und der nichtionische grenzflächenaktive Stoff oder die nichtionischen grenzflächenaktiven Stoffe als solche oder im Gemisch in die Zuführung der Kolonne (C1) gegeben und/oder am Kopf der Kolonnen (C1) und (C2) zugegeben werden, wobei sie in diesem Fall beispielsweise in eine Lösung auf Lösungsmittel- oder Acrylsäurebasis gegeben werden können, die die Inhibitoren enthält und dazu verwendet wird, die Inhibitoren am Kopf der Kolonnen zuzuführen.

## Claims

1. Process for purifying acrylic acid which is directed towards removing the polymer-type impurities in those places in the distillation columns in which they have a tendency to accumulate, and more particularly a process directed towards removing the polymeric impurities which are formed during a step for removing the aldehyde impurities from acrylic acid, according to which step the medium containing the acrylic acid to be purified is distilled by adding to the said medium at least one amino compound of hydrazine type and at least one polymerization inhibitor and by flushing away, at the foot of the distillation column, the heavy compounds formed by the said hydrazine-type amino compound(s) with the said impurities, **characterized in that** the distillation of the acrylic acid to be purified is also carried out in the presence of at least one compound of the nonionic surface agent category.

2. Process according to Claim 1, **characterized in that** the nonionic surface agents are chosen from:
· compounds of the polysaccharide ether and acetate family, preferably the ethers, and in particular those which are derived from cellulose or from starch, such as those represented by formula (I): 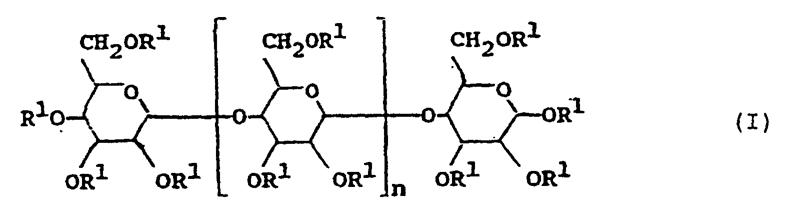 in which:
- the groups R¹ independently represent H; a C₁-C₄ alkyl group; CH₃CO-; 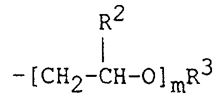 with R² = H, CH₃ or C₂H₅,
m = integer from 1 to 20 and R³ = H or C₁-C₄ alkyl or CH₃CO-; and
- n is an integer greater than 1, representing the number of chain units in a polymer chain;
· ethylene glycol and propylene glycol derivatives or ethers thereof, represented by formula (II): 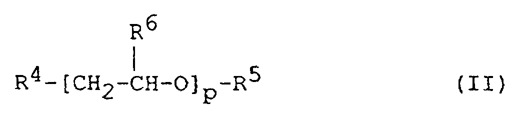 in which:
- R⁴ and R⁵ each independently represent HO- or R⁷O- or R⁷-C₆H₄O- with R⁷ representing a C₁-C₂₀ alkyl group;
- R⁶ represents H or CH₃; and
- p is an integer from 3 to 20;
· glycol ester derivatives, represented by formula (III): 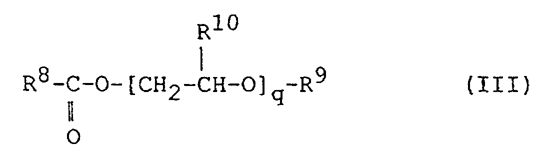 in which:
- R⁸ represents a C₈-C₂₀ alkyl group;
- R⁹ represents H or a C₈-C₂₀ alkyl group;
- R¹⁰ represents H or CH₃;
- q is an integer from 1 to 20;
· glyceryl ester derivatives, represented by formula (IV): 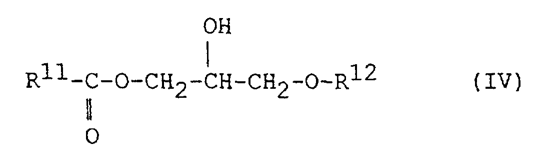 in which:
- R¹¹ represents a C₈-C₂₀ alkyl group; and
- R¹² represents H or 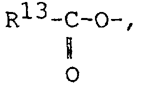 R¹³ representing a C₈-C₂₀ alkyl group;
· carboxylic amide derivatives, represented by formula (V): 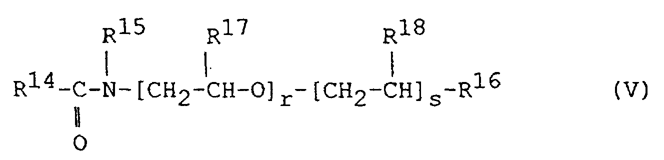 in which:
- R¹⁴ represents a C₈-C₂₀ alkyl group;
- R¹⁵ represents H or 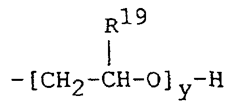 with R¹⁹ representing H or CH₃ and y being an integer from 1 to 5;
- R¹⁶ represents H or 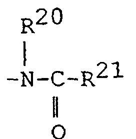 in which R²⁰ represents H
or 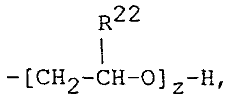 with R²² representing H or CH₃ and z is an integer from 1 to 5, and R²¹ is a C₈-C₂₀ alkyl;
- R¹⁷ and R¹⁸ each independently represent H or CH₃;
- r being equal to 0 or being an integer from 1 to 20; and
- s being equal to 0 or 1.

3. Process according to either of Claims 1 and 2, **characterized in that** the nonionic surface agent(s) is (are) sent into the flow containing the acrylic acid, alone or in solution or suspension in a solvent, such as acrylic acid, acetic acid, propionic acid, maleic acid, maleic anhydride or water, or in solution in a medium containing acrylic acid, for instance the solution rich in polymerization inhibitors which is used to inject these inhibitors into the top or into the feed of the distillation columns.

4. Process according to one of Claims 1 to 3, **characterized in that** the nonionic surface agent(s) is(are) added in a proportion of 10 - 10,000 ppm, preferably 10-1000 ppm, relative to the medium containing the acrylic acid to be purified.

5. Process according to one of Claims 1 to 4, **characterized in that** the polymerization inhibitors are chosen from phenolic derivatives, for instance hydroquinone and its derivatives, such as hydroquinone methyl ether; phenothiazine and its derivatives, such as methylene blue; metal thiocarbamates, such as copper dibutyldithiocarbamate; compounds containing nitroso groups, such as N-nitrosophenylhydroxylamine; quinones such as benzoquinone; paraphenylenediamine derivatives represented by the general formula (VI): 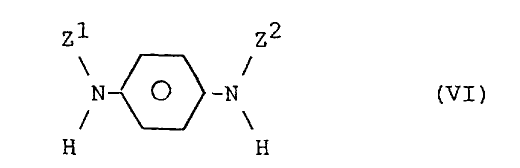 in which Z¹ and Z² each independently represent an alkyl, aryl, alkylaryl or arylalkyl radical.

6. Process according to one of Claims 1 to 5, **characterized in that** the polymerization inhibitor (s) is(are) added in a proportion of 5 - 10,000 ppm relative to the medium containing the acrylic acid to be purified.

7. Process according to one of Claims 1 to 6, **characterized in that** the hydrazine-type amino compound is chosen from hydrazine; hydrazine hydrate; alkylhydrazines such as cyclohexylhydrazine or hexadecylhydrazine; phenylhydrazine; 2-naphthylhydrazine; tolylhydrazine; p-nitrophenylhydrazine; 2,4-dinitrophenylhydrazine; glycine; guanidine; aminoguanidine; and salts thereof.

8. Process according to one of Claims 1 to 7, **characterized in that** the hydrazine-type amino compound(s) is (are) added in a proportion of 10 - 10,000 ppm relative to the medium containing the acrylic acid to be purifed.

9. Process according to one of Claims 1 to 8, **characterized in that** the hydrazine-type amino compound(s) is(are) added in an amount such that the molar ratio: hydrazine-type compound(s)/sum of the aldehydes present in the feed, is 0.5/1 - 10/1, in particular 1/1 - 5/1.

10. Process according to one of Claims 1 to 9, **characterized in that** it is carried out in batchwise mode, or alternatively in continuous mode, for example in the flow of acrylic acid to be purified feeding the monomer distillation column.

11. Process according to one of Claims 1 to 10, **characterized in that** the distillation is carried out in two successive steps:
- the first step, in a first column for removal of the head fraction (C1), fed with the flow of acrylic acid to be purified and receiving the hydrazine-type amino compound(s), in order to remove, at the head of the column, the light impurities and the water generated during the reaction of the hydrazine-type compound(s) with the aldehyde impurities; and
- the second step, in a second distillation column (C2), fed with the flow from the foot of the said column for removal of the head fraction (C1), in order to carry out, at the top of the column, the distillation of the pure acrylic acid, and, at the foot of the column, the removal of the heavy compounds, consisting in particular of the products of reaction of the aldehyde impurities with the hydrazine-type compounds, the polymerization inhibitor(s) and the nonionic surface agent(s),
the polymerization inhibitor(s) being added to the top of the column (C1) and to the top of the column (C2), and optionally into the feed of these two columns, the nonionic surface agent compound(s) being added, alone or as mixtures, into the feed of the column (C1) and/or into the top of the columns (C1) and (C2), in which case they can be injected, for example, in a solution based on solvent or on acrylic acid containing the inhibitors, which is used to send these inhibitors to the top of the said columns.
